# FASCICULE DE BREVET EUROPEEN

(11) **EP 3 958 985 B1**
(45) Date de publication et mention de la délivrance du brevet: **06.12.2023**
(21) Numéro de dépôt: 20754311.7
(22) Date de dépôt: 23.04.2020
(51) Int. Cl.: A61Q 19/04, A61Q 19/00, A61K 8/02, A61K 8/04, A61K 8/19, A61K 8/891, A61K 8/92

(54) **COMPOSE COMPRENANT UN AGENT FONCTIONNEL, ET PROCEDE DE FABRICATION AFFERENT**
VERBINDUNG MIT EINEM FUNKTIONELLEN MITTEL UND ENTSPRECHENDES VERFAHREN ZUR HERSTELLUNG
COMPOUND COMPRISING A FUNCTIONAL AGENT, AND RELATED MANUFACTURING METHOD

(30) Priorité: 26.04.2019 FR 1904459
(43) Date de publication de la demande: 02.03.2022
(73) Titulaire: Miyoshi Europe, 69800 Saint-Priest (FR)
(72) Inventeur: GOUGEON, Sébastien, 69800 SAINT-PRIEST (FR); DAUPHIN, Quentin, 69800 SAINT-PRIEST (FR); SELLAL, Laetitia, 69800 SAINT-PRIEST (FR); NICOLAS, Stephane, 69800 SAINT-PRIEST (FR); BOSMET, Pauline, 69800 SAINT-PRIEST (FR)
(74) Mandataire: Weber, Jean-François
(86) Numéro de dépôt international: PCT/FR2020/050689
(87) Numéro de publication internationale: WO 2020/217022

(56) Documents cités:
- FR-A1- 2 474 520
- GB-A- 906 973
- US-A- 4 235 641
- US-A- 4 464 203
- US-A1- 2002 151 639
- US-A1- 2014 005 316
- US-B1- 6 316 547
- DATABASE WPI Week 201501 Thomson Scientific, London, GB; AN 2014-W56613 XP002796364, & CN 104 059 273 A (BLUESTAR CHENGDU NEW MATERIAL CO LTD) 24 septembre 2014 (2014-09-24)
- DATABASE WPI Week 199429 Thomson Scientific, London, GB; AN 1994-237470 XP002796365, & JP H06 93201 A (TOKYO INK KK) 5 avril 1994 (1994-04-05)

## Description

### DOMAINE TECHNIQUE

La présente invention se rapporte au domaine technique général des substances et composés utilisés dans le domaine de la formulation de compositions cosmétiques et/ou dermatologiques, en particulier du genre composé comportant un agent fonctionnel, par exemple de type pigment.

La présente invention concerne plus particulièrement un composé comprenant au moins 50% en poids d'un agent fonctionnel.

La présente invention concerne également un procédé de fabrication d'un tel composé.

La présente invention concerne enfin un procédé de fabrication d'une composition cosmétique et/ou dermatologique.

### TECHNIQUE ANTERIEURE

La fabrication des compositions cosmétiques et/ou dermatologiques nécessite habituellement de réunir un certain nombre de substances et composés pour les mélanger en un matériau homogène, c'est-à-dire qui présente une répartition régulière des différents composés et substances, et qui est stable, à savoir notamment sans séparation de phases dans le temps, par sédimentation ou crémage par exemple, ou autre phénomène indésirable tel que la coalescence ou la floculation.

À cette fin, il est connu de recourir à des pigments solides qui sont broyés en fines particules par des unités de broyage, avant d'être intégrés dans une composition cosmétique pour la colorer ou renforcer son aspect mat ou brillant. La mise en oeuvre de ces pigments broyés, bien que donnant globalement satisfaction dans leur utilisation finale, n'en présentent pas moins certains inconvénients.

Un des inconvénients important de l'utilisation de ces pigments broyés dans la réalisation d'une composition cosmétique réside dans le caractère pulvérulent de ces derniers, lequel rend le stockage, le transport et la manipulation de ces pigments broyés complexes, coûteux, salissants, et même polluants, tout en présentant un risque non négligeable concernant la sécurité des êtres vivants et des installations, ainsi que des difficultés importantes relativement au nettoyage et/ou à l'élimination de ces pigments broyés. De manière générale, plus le broyage des pigments est fin et donc les particules de pigment petites, plus leur gestion se révèle complexe. Par ailleurs, l'état poudreux de ces pigments leur confère une grande surface spécifique (surface réelle / faible masse) entraînant elle-même une importante réactivité, et donc notamment un risque accru de dégradation dans le temps, voire même un risque d'accident, notamment de type explosion.

De plus, les unités de broyage sont bien souvent spécialisées et très coûteuses, tandis que le processus de broyage lui-même peut se révéler particulièrement long et complexe selon la nature et la taille désirée des particules de pigment broyé, qui peuvent varier selon des exigences par exemple esthétiques, organoleptiques et/ou qualitatives. Ces unités de broyage, dans la plupart des cas, du fait de leur coût et de leur complexité, ne sont pas dédiées spécifiquement au broyage spécifique d'un pigment, et nécessitent donc des réglages pour broyer un pigment selon d'autres spécifications, telle une granulométrie différente, ou pour broyer d'autres types de pigments. Ainsi, ces unités de broyage réalisent généralement différents « *batchs* » ou lots de plusieurs types et tailles pigments broyés qu'il faut ensuite stocker, transporter et utiliser au niveau de différentes unités de fabrication de compositions cosmétiques. Il en découle d'une part une multiplicité de lots de pigments pulvérulents difficile à gérer (stockage, transport, manipulation notamment lors du mélange avec d'autres substances pour réaliser la formulation d'une composition cosmétique, évitement des mélanges de pigments différents), et d'autre part que les unités de fabrication de compositions cosmétiques sont presque toujours distinctes et distantes des unités de broyage, ce qui complique d'autant plus la gestion (notamment le transport) des pigments broyés.

En définitive, l'intégration, telle qu'elle est actuellement pratiquée, des pigments broyés dans les compositions cosmétiques, présente une insuffisance en matière de facilité de mise en oeuvre, de stockage, de transport, de sécurité, et de coûts.

Par ailleurs, GB-A-906 973 décrit une composition pour colorer des matières plastiques qui comprend 40 à 70% de colorant pigmentaire et 60 à 30% d'une cire de polyéthylène.

### EXPOSE DE L'INVENTION

L'invention se propose par conséquent de remédier aux différents inconvénients exposés précédemment en proposant un nouveau composé qui non seulement s'avère capable d'être facilement intégrée à une composition cosmétique et/ou dermatologique, mais présente en outre une grande facilité de mise en oeuvre en ce qui concerne sa gestion et notamment son transport, son stockage et sa manipulation.

Un autre objet de l'invention vise à proposer un nouveau composé de conception extrêmement simple et bon marché.

Un autre objet de l'invention vise à proposer un nouveau composé particulièrement aisé à manipuler.

Un autre objet de l'invention vise à proposer un nouveau composé particulièrement aisé à transporter et à stocker.

Un autre objet de l'invention vise à proposer un nouveau composé facile à intégrer efficacement à des compositions cosmétiques et/ou dermatologiques.

Un autre objet de l'invention vise à proposer un nouveau composé dont la conception lui permet d'obtenir une bonne répétabilité en tant que composant pour la formulation de compositions cosmétiques et/ou dermatologiques.

Un autre objet de l'invention vise à proposer un nouveau composé dont la conception lui permet d'être utilisé dans une très large gamme d'applications, notamment pour une très large variété de compositions cosmétiques et/ou dermatologiques.

Un autre objet de l'invention vise à proposer un nouveau composé présentant un risque minime vis-à-vis de la sécurité des êtres vivants et qui ne dégrade pas les installations.

Un autre objet de l'invention vise à proposer un nouveau composé de fabrication aisément industrialisable, mettant en oeuvre un nombre minimum de substances différentes.

Un autre objet de l'invention vise à proposer un nouveau composé qui ne se dégrade que très peu voire pas du tout dans le temps.

Un autre objet de l'invention vise à proposer un procédé de fabrication d'un composé dont la mise en oeuvre est simple, facile et bon marché, et présente une bonne répétabilité et une bonne sécurité pour l'utilisateur.

Un autre objet de l'invention vise à proposer un procédé de fabrication d'une composition cosmétique et/ou dermatologique dont la mise en oeuvre est rapide, facile à contrôler et aisée, tout en garantissant des conditions de sécurité optimum.

Les objets assignés à l'invention sont atteints à l'aide d'un composé comprenant au moins 50% en poids d'un agent fonctionnel, caractérisé en ce qu'il comprend également une matrice cireuse au sein de laquelle ledit agent fonctionnel est dispersé, ledit composé étant solide à température ambiante et destiné à être dispersé par fluidification et/ou solubilisation au sein d'une composition cosmétique et/ou dermatologique pour y intégrer ledit agent fonctionnel, ledit agent fonctionnel étant formé de particules solides ayant subi un traitement de surface entraînant une diminution de la capacité d'absorption de matrice cireuse par lesdites particules solides traitées, comparativement à des particules solides de même nature non traitées.

Les objets assignés à l'invention sont également atteints à l'aide d'un procédé de fabrication d'un composé comprenant au moins 50% en poids d'un agent fonctionnel, caractérisé en ce qu'il comprend au moins :
- une étape de chauffage d'une matrice cireuse afin d'obtenir une matrice fluide,
- une étape de traitement de particules solides au cours de laquelle ces dernières subissent un traitement de surface entraînant une diminution de la capacité d'absorption de matrice fluide par les particules solides traitées, comparativement à des particules solides de même nature non traitées, lesdites particules solides traitées formant ledit agent fonctionnel,
- une étape de dispersion dudit agent fonctionnel au sein de ladite matrice fluide, ledit composé étant solide à température ambiante et destiné à être dispersé par fluidification et/ou solubilisation au sein d'une composition cosmétique et/ou dermatologique pour y intégrer ledit agent fonctionnel.

Les objets assignés à l'invention sont en outre atteints à l'aide d'un procédé de fabrication d'une composition cosmétique et/ou dermatologique comprenant une étape de redispersion au cours de laquelle un composé tel que mentionné ci-avant est ajouté à puis mélangé avec un ou plusieurs composants acceptables en cosmétique et/ou en dermatologie.

### DESCRIPTIF SOMMAIRE DES DESSINS

D'autres particularités et avantages de l'invention apparaîtront et ressortiront plus en détail à la lecture de la description faite ci-après, en référence aux dessins annexés, donnés à titre d'exemples purement illustratifs et non limitatifs, dans lesquels :
La figure 1 illustre de façon schématique, selon une vue de côté, un exemple de composé selon l'invention, qui se présente en l'espèce sous la forme d'un bâtonnet obtenu par extrusion.
La figure 2 illustre de façon schématique, à l'échelle microscopique ou nanométrique, un détail du composé de la figure 1.
La figure 3 illustre de façon schématique, selon une vue de côté, une partie d'un exemple d'équipement de production permettant la mise en oeuvre d'un procédé de fabrication de composé selon l'invention, selon un mode particulier de réalisation mettant en oeuvre une étape d'extrusion.

### MEILLEURE MANIERE DE REALISER L'INVENTION

Comme illustré aux figures, l'invention concerne un composé 1. Ledit composé 1 est donc préférentiellement formé de plusieurs éléments différents, plus précisément d'un mélange relativement homogène et stable de plusieurs substances, matériaux, matières différentes ensembles, tous avantageusement acceptables sur le plan cosmétique et/ou dermatologique, c'est-à-dire convenables pour être intégrés au sein d'une composition cosmétique et/ou dermatologique. Le composé 1 présente donc avantageusement un niveau de toxicité très faible voire nul au regard de l'utilisation d'une composition cosmétique et/ou dermatologique. Ainsi, selon l'invention, le composé 1 comprend au moins 50% en poids d'un agent fonctionnel. Selon un mode de réalisation préférentiel, le composé 1 comprend (strictement) plus de 50%, de préférence (strictement) plus de 60%, plus préférentiellement au moins 70%, plus préférentiellement encore au moins 80%, voire même encore plus préférentiellement au moins 85%, plus préférentiellement encore au moins 90%, en poids, dudit agent fonctionnel. Ledit agent fonctionnel est avantageusement choisi de manière appropriée, de façon à former un élément acceptable pour être intégré au sein d'une composition cosmétique et/ou dermatologique. Ainsi, une proportion élevée d'agent fonctionnel au sein du composé 1 est particulièrement avantageuse lorsque ce dernier constitue un produit intermédiaire (ou éventuellement quasi-fini) destiné à être redispersé avec d'autres composants pour former un produit cosmétique et/ou dermatologique fini, comme cela sera décrit plus en détail ci-après. En effet, ledit composé 1 sert avantageusement de « *support* » ou de « *véhicule* » intermédiaire pour l'agent fonctionnel, et il est donc préférable que la proportion d'agent fonctionnel au sein du composé 1 soit la plus élevée possible d'une part pour que la formulation finale (c'est-à-dire le produit cosmétique et/ou dermatologique fini) ne soit pas ou que très peu tributaire de la nature des substances autres que ledit agent fonctionnel au sein dudit composé 1, et d'autre part pour des raisons évidentes de coûts, de stockage et de transport, un composé intermédiaire présentant une faible teneur en agent fonctionnel devant par définition être utilisé en plus grande quantité et donc occupant un volume plus grand pour un poids globalement plus important pour une quantité d'agent fonctionnel donnée, tandis que les substances autres que ledit agent fonctionnel au sein dudit composé 1, étant en plus grandes proportions, représentent un coût important qui n'est de surcroît pas valorisé dans la formulation finale (voire viennent « *parasiter* » ladite formulation finale, ou cosmétique et/ou dermatologique fini) Préférentiellement, ledit agent fonctionnel est destiné à remplir une ou plusieurs fonctions bien spécifiques au sein d'une composition cosmétique et/ou dermatologique. Par exemple, ledit agent fonctionnel est destiné à former un filtre solaire physique, notamment au sein d'une composition cosmétique et/ou dermatologique de type crème solaire, c'est-à-dire qu'il est destiné à former une barrière physique, de préférence opaque, entre les rayons ultraviolets du soleil et la peau d'un utilisateur de ladite composition cosmétique et/ou dermatologique.

Selon l'invention, ledit agent fonctionnel est formé de particules solides 2, c'est-à-dire qui sont de préférence solides au moins à température ambiante, et même solides à des températures plus élevées, comme par exemple entre 30 et 90°C. Préférentiellement, lesdites particules solides sont insolubles dans l'eau. Préférentiellement, lesdites particules solides présentent une taille moyenne comprise entre 10 nm et 500 µm, de préférence entre 100 nm et 100 µm. Ladite taille moyenne désigne donc de préférence la granulométrie moyenne desdites particules 2, et en particulier leur diamètre moyen ou bien encore une dimension maximale moyenne desdites particules, pouvant être mesuré(e) notamment à l'aide d'un échantillon seulement desdites particules.

Par exemple, lesdites particules solides 2 comprennent au moins des pigments organiques et/ou minéraux (c'est-à-dire inorganiques), des charges organiques et/ou minérales, des matériaux composites et/ou biocomposites, ou un mélange de ceux-ci. Selon un mode de réalisation particulier, lesdites particules solides 2 sont principalement, voire totalement, formées de pigments minéraux, lesquels peuvent être constitués par exemple de dioxyde de titane, d'oxyde(s) de fer, d'oxyde(s) de chrome, de pigment(s) d'outremer (bleu ou violet d'outremer par exemple), d'oxyde(s) d'étain et/ou d'oxyde de zinc. Lesdits pigments organiques sont par exemple constitués de noir de carbone et/ou de laques organiques.

Lesdites charges minérales sont par exemple constituées de talc, de mica, de séricite, de kaolin, de silice, d'hydroxyapatite, de sulfate de baryum, de perlite, de carbonate de calcium, et/ou de borosilicates. Lesdites charges organiques sont par exemple constituées d'amidon, de cellulose, de polymères d'origine naturelle ou synthétique, et/ou de polymères et résines siliconés. Lesdits composites et/ou biocomposites incluent par exemple une ou plusieurs nacres. Lorsque lesdites particules solides sont des pigments, l'agent fonctionnel est par exemple destiné à colorer une formulation cosmétique et/ou dermatologique, ou encore à protéger la peau du soleil (cas de la crème solaire évoqué précédemment, les particules solides 2 formant notamment un filtre solaire). Lorsque lesdites particules solides 2 sont des charges, l'agent fonctionnel peut par exemple être destiné à modifier les propriétés organoleptiques d'une formulation cosmétique et/ou dermatologique.

Toujours selon l'invention, le composé 1 comprend également une matrice cireuse 3 au sein de laquelle ledit agent fonctionnel est dispersé. En d'autres termes, ledit agent fonctionnel, et plus précisément lesdites particules solides 2, est/sont avantageusement mélangé(es) avec ladite matrice cireuse 3 et éparpillé(es) au sein de cette dernière, de préférence de façon homogène, c'est-à-dire selon une répartition volumique régulière. Avantageusement, au sein du composé 1, la matrice cireuse 3 et lesdites particules solides 2 forment, d'un point de vue macroscopique, une seule phase, et il est impossible de différencier à l'œil nu la matrice cireuse 3 desdites particules solides 2.

Par exemple, ladite matrice cireuse 3 comprend au moins une cire naturelle, minérale et/ou synthétique, un corps gras solide à température ambiante issu du fractionnement d'une ou plusieurs huile(s) naturelle(s) et/ou synthétique(s), une résine naturelle et/ou synthétique, une cire de silicone ou de polymère de silicone, un matériau organosilicié (ou organosilicique) ou bien un mélange de ceux-ci. Par exemple, ledit matériau organosilicié (ou une pluralité de différents matériaux organosiliciés) fait partie de la famille des silsesquioxanes, lesquels présentent la formule générale (RSiO_{3/2})ₙ, R représentant un groupe carboné notamment de type alcane, alcène, aryle ou arylène, tandis que de manière usuelle Si représente un atome de silicium, et O représente un atome d'oxygène, n représentant un nombre entier généralement supérieur à 1, par exemple compris entre 3 et 20, tel qu'égal à 8. Ledit matériau organosilicié est avantageusement un polymère de silsesquioxane. Selon un mode de réalisation particulier de l'invention, ladite matrice cireuse 3 comprend du polyméthylsilsesquioxane, et est par exemple principalement formée par ce dernier. Le polyméthylsilsesquioxane est un type de matériau organosilicié particulièrement efficace lorsqu'il est utilisé en tant que matrice cireuse 3. Selon d'autres modes particuliers de réalisation, la matrice cireuse 3 comprend, principalement (en poids) ou non, du cyclopentasiloxane, au moins une alkyl(en C30 à C45)-méthicone et/ou des esters hydrogénés tels que les esters hydrogénés d'huiles végétales, par exemple un ester hydrogéné d'huile d'olive de nom INCI « *Hydrogenated olive oil stearyl esters* ». Bien évidemment, ladite matrice cireuse 3 peut éventuellement être formée d'un mélange de plusieurs substances, notamment parmi celles mentionnées ci-avant. Ledit matériau organosilicié est préférentiellement un matériau organosiliconé. De façon préférentielle, ladite matrice cireuse 3 est sensiblement solide à température ambiante, et notamment en-dessous de 30°C, de préférence en-dessous de 45°C, plus préférentiellement en-dessous de 55°C, de façon plus préférentielle encore en-dessous de 65°C. A l'inverse, ladite matrice cireuse 3 est avantageusement sensiblement fluide à une température supérieure à la température ambiante, et par exemple une température égale ou supérieure à 30°C, de préférence 45°C, de manière plus préférentielle 55°C, et de manière encore plus préférentielle 65°C. Ainsi, de manière avantageuse, ladite matrice cireuse 3 présente un point de fusion et/ou un point de ramollissement compris entre 30 et 150°C inclus, plus préférentiellement entre 45 et 130°C inclus, plus préférentiellement encore entre 55 et 130°C inclus. Dans certains modes de réalisation de l'invention, ladite matrice cireuse 3 présente un point de fusion et/ou un point de ramollissement compris entre 55 et 150°C inclus, par exemple entre 55 et 95°C inclus, ou entre 95 et 150°C inclus. Ladite matrice cireuse 3 présente par exemple une densité comprise environ entre 0,80 et 1,5, de préférence entre 0,9 et 1,3, par exemple de 1,24 environ dans le cas où la matrice cireuse 3 est principalement, voire totalement, formée de polyméthylsilsesquioxane. Par exemple, en particulier lorsque ladite matrice cireuse 3 est principalement, voire totalement, formée de polyméthylsilsesquioxane, son point de ramollissement est avantageusement compris entre 55 et 150°C, voire entre 75 et 90°C. Ladite matrice cireuse 3 est de préférence formée par un matériau présentant des caractéristiques physiques générales similaires ou équivalentes à celles des cires, et en particulier un matériau qui se fluidifie significativement au-dessus d'une certaine température, c'est-à-dire un matériau qui perd sa solidité ou passe d'une viscosité très élevée à une viscosité bien plus basse lorsqu'il est chauffé à une certaine température ou au-dessus. Par exemple, ladite matrice cireuse 3 est formée par un matériau sensiblement thermofusible et/ou thermoplastique, et se ramollit de manière significative et/ou fond lorsqu'elle est chauffée à ou au-dessus d'une certaine température, par exemple 65°C. Ladite matrice cireuse 3 est donc préférentiellement solide à température ambiante et devient de préférence sensiblement fluide lorsqu'elle est portée à une certaine température au-dessus de la température ambiante, notamment au-dessus de son point de fusion et/ou de ramollissement. La matrice cireuse 3 peut avantageusement être formée, à température ambiante, de granulés solides distincts les uns des autres, c'est-à-dire difficilement compactables ou non compactables par pression mécanique. Il est particulièrement avantageux que ladite matrice cireuse 3 soit solide à température ambiante, car c'est préférentiellement ce qui va permettre au composé 1 de rester lui-même sous forme solide à température ambiante, ladite matrice cireuse 3 déterminant éventuellement le comportement physique du composé 1 en particulier lorsque ledit agent fonctionnel est lui-même formé par une substance solide à température ambiante.

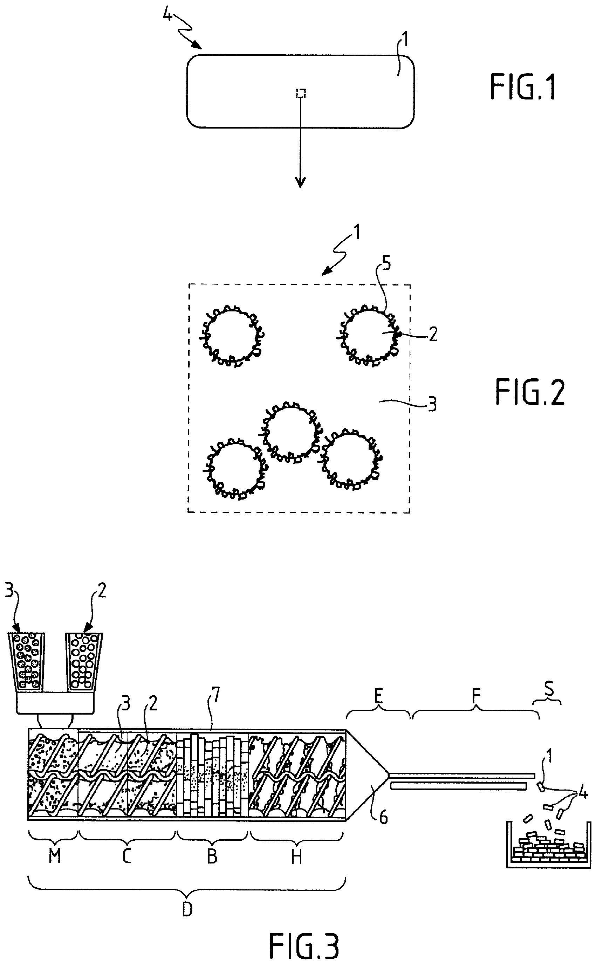

Selon l'invention, ledit composé 1 est solide à température ambiante et destiné à être dispersé par fluidification et/ou solubilisation au sein d'une composition cosmétique et/ou dermatologique pour y intégrer ledit agent fonctionnel. Avantageusement, le composé 1 présente à température ambiante ou encore en-dessous de 30°C voire en-dessous de 45°C par exemple, une consistance relativement ferme, non flasque, c'est-à-dire une tenue mécanique minimum, le différenciant nettement d'un fluide et notamment d'un liquide. Par exemple, ledit composé 1 se présente sous la forme de granulés, par exemple des pastilles ou des bâtonnets 4, obtenus par extrusion, comme illustré aux figures 1 et 3. Ledit composé 1 est de préférence destiné à être intégré ou incorporé au sein d'une composition cosmétique et/ou dermatologique en cours de formulation (c'est-à-dire de fabrication). En d'autres termes, le composé 1 est avantageusement destiné à être mélangé, de préférence de manière homogène, avec des composants acceptables d'un point de vue cosmétique et/ou dermatologique, pour former une composition cosmétique et/ou dermatologique. De manière avantageuse, l'incorporation du composé 1 au sein d'une composition cosmétique et/ou dermatologique est, comme on le verra plus en détail par la suite avec le procédé, réalisée par l'une et/ou l'autre des actions suivantes :
- à l'aide d'une fluidification, obtenue par chauffage, dudit composé 1, c'est-à-dire une perte du caractère solide dudit composé 1, généralement via un ramollissement ou une fusion (au moins partielle, puisque les particules solides ne se fluidifient avantageusement pas) de ce dernier et/ou de la matrice cireuse 3 ; le mélange prévu du composé 1 fluidifié (« *à chaud* ») avec des composants acceptables d'un point de vue cosmétique et/ou dermatologique pour former une composition cosmétique et/ou dermatologique peut être facilité car lesdits composants sont avantageusement principalement sous forme fluide eux aussi (« *à chaud* » eux aussi ou non) ;
- à l'aide d'une solubilisation dudit composé 1, par un ou plusieurs composants acceptables d'un point de vue cosmétique et/ou dermatologique, pour former une composition cosmétique et/ou dermatologique, ces composants étant de préférence principalement sous forme fluide, au moins l'un de ces composants étant capable de solubiliser ledit composé 1, notamment même lorsque ce dernier est « *à froid* » (c'est-à-dire à température ambiante, notamment aux alentours de 20°C ou en-dessous de 30°C), ledit composant 1 pouvant bien naturellement éventuellement être « *à chaud* » (dans ce cas-là, on parlera plutôt de mélange par fluidification comme décrit ci-avant, une combinaison de fluidification et solubilisation étant évidemment possible). Selon un exemple de réalisation non limitatif donné uniquement à titre illustratif, le composé 1 comprend une matrice cireuse 3 formée principalement d'une cire de silicone, laquelle est facilement dissoute par des composants acceptables d'un point de vue cosmétique et/ou dermatologique notamment de type huile, ledit composé 1 étant alors également avantageusement facilement soluble dans lesdits composants acceptables d'un point de vue cosmétique et/ou dermatologique pour former une composition cosmétique et/ou dermatologique.

Ladite composition cosmétique et/ou dermatologique peut être destinée directement à un utilisateur, ou former un produit intermédiaire lui-même destiné à intégrer un produit cosmétique et/ou dermatologique lui-même final, destiné à un utilisateur, ou intermédiaire.

Ledit composé 1, résultant avantageusement du mélange de l'agent fonctionnel, c'est-à-dire des particules 2, et de la matrice cireuse 3, ne présente de préférence pas de caractère pulvérulent, mais un caractère cohésif et facilement « *redispersible* » au sein d'une composition cosmétique et/ou dermatologique en cours de formulation sous forme fluide. A froid, c'est-à-dire à température ambiante, le composé 1 constitue donc avantageusement une dispersion solide, à la manière d'un ensemble de particules solides 2 dispersées au sein de la matrice cireuse 3 elle aussi solide à froid. Préférentiellement, une telle configuration permet en particulier de s'affranchir des contraintes liées au caractère souvent naturellement pulvérulent desdites particules solides 2 seules. Ainsi, de manière préférentielle, le composé 1 est sensiblement plus facile à gérer, c'est-à-dire à stocker, transporter et manipuler, que lesdites particules 2 seules, notamment du fait que le composé 1 se trouve par exemple, à température ambiante, sous la forme de petits solides (par exemple des granulés de type pastille ou bâtonnet 4), cohérents, relativement inertes, et facilement manipulables, tandis que lesdites particules 2, lorsqu'elles sont seules, présentent au contraire souvent un caractère extrêmement fractionné, poudreux, avec une forte réactivité et un caractère dispersible élevé dans l'air. Le composé 1 est avantageusement formé, à température ambiante, de solides distincts les uns des autres, de préférence de taille réduite tels que des granulés, par exemple des bâtonnets des pastilles 4, lesdits solides distincts étant préférentiellement difficilement ou non compactables par pression mécanique. Ainsi, ledit composé 1 est facile à stocker et aisément transportable, sous forme desdits (petits) solides distincts entassés en vrac dans des contenants, par exemple des sacs, de plusieurs kilogrammes ou plusieurs dizaines de kilogrammes (au moins), sans que lesdits solides distincts ne se solidarisent entre eux, par exemple par agglomération, par coalescence, par écrasement, ou par sédimentation. En d'autres termes, ledit composé 1 est de préférence facile à stocker et transporter sous forme desdits solides distincts les uns des autres, et qui restent distincts les uns des autres pendant le transport et/ou le stockage, même lorsqu'il(s) est/sont effectué(s) à l'aide de contenants du genre sac pesant chacun, avec leur contenu (formé par lesdits solides distincts), plusieurs kilogrammes ou dizaines de kilogrammes. Les solides distincts, formés de composé 1, restent préférentiellement aisément manipulables même après avoir été stockés et/ou transportés au sein desdits contenants, et ce grâce à l'absence de solidarisation desdits solides distincts même lorsqu'ils sont entassés en vrac, lesdits solides distincts conservant leur caractère unitaire respectif en dépit de l'entassement. Bien évidemment, le transport décrit ci-avant est de préférence réalisé à température ambiante ou une température légèrement plus chaude que la température ambiante, mais qui reste avantageusement en-dessous du point de fusion et/ou de ramollissement de ladite matrice cireuse 3. Ainsi, le composé 1 est non seulement pratique à transporter et stocker, mais également à quantifier lorsqu'il faut le disperser au sein d'une composition cosmétique et/ou dermatologique, lesdits solides distincts, et notamment les granulés (par exemple des pastilles ou des bâtonnets 4). Ceci permet notamment de quantifier aisément la quantité d'agent fonctionnel à obtenir dans la composition cosmétique et/ou dermatologique finale, ce qui implique une excellente répétabilité quant à la formulation de cette dernière grâce au composé 1.

Selon l'invention, lesdites particules solides 2 ont subi un traitement de surface entraînant une diminution de la capacité d'absorption de matrice cireuse 3 par lesdites particules solides 2 traitées, comparativement à des particules solides de même nature non traitées. En d'autres termes, lesdites particules solides 2, qui forment l'agent fonctionnel du composé 1, ont avantageusement subi un traitement de surface permettant de les « *saturer* » plus rapidement en matrice cireuse 3, c'est-à-dire à l'aide d'une quantité moindre de matrice cireuse 3 par rapport à une quantité de particules solides 2 considérée. Par exemple, la limite d'absorption (ou « *saturation* ») de matrice cireuse 3 par une quantité de particules solides 2 (traitées ou non) est testée de la façon suivante : une quantité (plus que nécessaire) de matrice cireuse 2 est chauffée pour être fluidifiée, par exemple chauffée à 65°, et est ajoutée goutte à goutte à une quantité considérée de particules solides 2 (traitées ou non), qui sont de préférence soumises en même temps à une action de mélange ou de trituration ; le mélange particules solides 2 / gouttes de matrice cireuse 3 mélangé ou trituré va d'abord former une pâte extrêmement visqueuse voire solide ou quasi-solide, à la manière d'une pâte à modeler par exemple, puis va, à l'ajout d'une goutte supplémentaire de matrice cireuse 3, voir sa viscosité chuter de façon significative et / ou passer d'un état solide ou quasi-solide à un état fluide ou quasi-fluide (liquide ou ramolli notamment). Ledit traitement de surface permet donc avantageusement de diminuer la quantité de matrice cireuse 3 fluidifiée à ajouter à une quantité considérée de particules solides 2 pour que leur mélange perde sa consistance, c'est-à-dire passe d'un état très visqueux, solide ou quasi-solide, à un état significativement moins visqueux, fluide ou quasi-fluide. Les traitements de surface permettant d'entraîner une diminution de la capacité d'absorption de matrice cireuse sont connus en tant que tels et se déclinent selon de multiples alternatives, l'invention n'étant évidemment pas limitée à un traitement particulier. A titre d'exemple non limitatif, le traitement de surface des particules solides 2 mis en oeuvre dans le cadre de l'invention peut reposer sur un traitement à base d'acides aminés, par exemple des acides N-acylaminés tels que l'acide N-acyl-L-glutamique, et être avantageusement conforme à l'enseignement du brevet US-4,606,914. Par exemple, le traitement de surface repose sur un traitement à base d'acides aminés se présentant sous la forme de sels métalliques d'acide N-acylaminé, tels qu'un sel réalisé à partir de myristoyl glutamate de sodium et d'hydroxyde d'aluminium. Bien évidemment, d'autres traitements de surface conformes à l'invention sont envisageables, et le traitement de surface peut par exemple reposer sur : un traitement mettant en oeuvre du stéaroyl glutamate de disodium, ainsi qu'avantageusement du dimyristate d'aluminium et du triéthoxycaprylylsilane ; un traitement mettant en oeuvre un alkoxysilane tel que le triéthoxycaprylylsilane ; un traitement conforme à l'enseignement du brevet US-6482441, un traitement mettant en oeuvre un polymère silicone tel que le diméthicone, un traitement mettant en oeuvre un acide gras, un traitement mettant en oeuvre un ou des phospholipides, un traitement mettant en oeuvre un ester d'huile d'olive hydrogénée. Lesdites particules solides 2 ayant subi un traitement de surface sont par exemple formées de pigments bruts (de préférence plus de 90% en poids), de myristoyl glutamate de sodium (de préférence moins de 10% en poids) et d'un hydroxyde d'aluminium (de préférence moins de 10% en poids), selon un exemple purement illustratif et non limitatif. Le traitement de surface permettant d'entraîner une diminution de la capacité d'absorption de matrice cireuse peut en outre être formé par une combinaison des différents traitements listés ci-avant. Les traitements de surface permettant d'entraîner une diminution de la capacité d'absorption de matrice cireuse permettent également avantageusement d'augmenter le caractère hydrophobe des particules solides 2 traitées, et/ou d'améliorer leur dispersibilité en phase huile, par rapport à des particules solides de même nature non traitées.

De façon avantageuse, ledit agent fonctionnel forme (strictement) plus de 50%, de préférence plus de 60%, plus préférentiellement au moins 70%, plus préférentiellement encore au moins 80%, encore plus préférentiellement au moins 85% ou même au moins 90%, en poids, dudit composé 1. Ainsi, de manière préférentielle, au moins 70%, de préférence au moins 80%, voire au moins 85%, en poids, dudit composé 1, est formé par lesdites particules solides 2. Une telle proportion massique élevée en agent fonctionnel (et donc en particules 2) est avantageusement rendue possible grâce audit traitement de surface. Par corollaire, ladite matrice cireuse forme préférentiellement entre 5 et 25%, de préférence entre 7 et 22%, plus préférentiellement entre 15 et 20%, en poids, du composé 1. En résumé, de façon avantageuse, grâce au traitement de surface, une quantité relativement faible de matrice cireuse 3 est suffisante pour servir de support à une quantité relativement importante de particules solides 2.

Préférentiellement, le composé 1, lorsqu'il est fluidifié et/ou solubilisé, et par extension l'agent fonctionnel qu'il contient (et plus précisément lesdites particules solides 2), peu(ven)t être dispersé(s) ou mélangé(s) de manière homogène et stable au sein d'une composition cosmétique et/ou dermatologique. En résumé, grâce au principe général de l'invention, et notamment au traitement de surface, il est possible, de manière avantageuse, non seulement d'obtenir un composé 1 facilement manipulable comprenant une quantité élevée d'agent fonctionnel (au moins 50%, de préférence au moins 80 ou même plus préférentiellement encore au moins 85% en poids) et une faible quantité de matrice cireuse 3, mais également de réaliser, à terme, une dispersion aisée du composé 1 au sein d'une composition cosmétique et/ou dermatologique en cours de fabrication pour y intégrer ledit agent fonctionnel. La matrice cireuse 3, et par extension le composé 1, fait avantageusement office de support pour l'agent fonctionnel avant sa redispersion au sein d'une composition cosmétique et/ou dermatologique. De préférence, la redispersion du composé 1 peut se faire par fluidification et donc chauffage du composé 1 jusqu'à le rendre fluide, pour le mélanger plus facilement à des composants acceptables d'un point de vue cosmétique et/ou dermatologique eux-mêmes fluides, ou par solubilisation du composé 1, de préférence sous forme de petits solides (des granulés notamment, par exemple des pastilles ou des bâtonnets 4), éventuellement à froid (c'est-à-dire sans chauffage), au sein de composants acceptables d'un point de vue cosmétique et/ou dermatologique également fluides.

De plus, la quantité relativement faible, ou limitée, de matrice cireuse 3 au sein dudit composé 1, ainsi que la nature de cette dernière, permet avantageusement audit composé 1 de pouvoir être intégré à une très large gamme de compositions cosmétiques et/ou dermatologiques. En d'autres termes, la conception du composé 1 permet de manière avantageuse d'intégrer ledit agent fonctionnel qu'il contient au sein d'une composition cosmétique et/ou dermatologique, sans contrainte ou pratiquement sans contraintes vis-à-vis des autres composants de cette dernière, c'est-à-dire sans avoir besoin de modifier ces autres composants formant ladite composition cosmétique et/ou dermatologique pour les adapter audit composé 1. En résumé, le composé 1 de l'invention est avantageusement susceptible d'être intégré à une grande variété de compositions cosmétiques et/ou dermatologiques.

De préférence, ladite matrice cireuse 3 est liposoluble et/ou insoluble ou quasiment insoluble dans l'eau. Ainsi, la matrice cireuse 3, qui présente avantageusement des caractéristiques physiques proches de ou similaires à celles des cires naturelles ou synthétiques (et notamment des cires silicones), présente préférentiellement un caractère lipophile, liposoluble et/ou hydrophobe. La matrice cireuse 3 se dissout en particulier dans des phases huiles utilisées couramment utilisées dans la fabrication des compositions cosmétiques et/ou dermatologiques. De façon avantageuse, en raison de sa faible quantité relative au sein du composé 1, la matrice cireuse 3 est destinée à intégrer relativement aisément la composition cosmétique et/ou dermatologique dans laquelle le composé est destiné lui-même à être intégré, de préférence sans modifier significativement les propriétés physiques et/ou chimiques de ladite composition, et ce d'autant plus facilement lorsque ladite composition présente une phase grasse ou huile (et/ou lipophile) éventuellement au sein d'une émulsion du type eau dans huile, par exemple de type eau dans silicone, ou alternativement de type huile dans eau.

Ledit composé 1 peut éventuellement être destiné à être redispersé pour faire partie d'une composition cosmétique et/ou dermatologique ne comprenant qu'une phase anhydre, de préférence de type grasse/huile/silicone, ladite composition étant par exemple un baume, un rouge à lèvre.

De manière avantageuse, ledit traitement de surface permet également d'augmenter le caractère lipophile et/ou hydrophobe des particules solides 2 traitées, comparativement à des particules solides 2 de même nature non traitées. Ainsi, le traitement de surface confère auxdites particules solides 2 ou renforce chez ces dernières une affinité chimique pour les graisses et matières grasses et assimilées dont fait avantageusement partie ladite matrice cireuse 3. Une telle caractéristique permet notamment d'obtenir une meilleure dispersion des particules solides 2 au sein de ladite matrice cireuse 3 et / ou une meilleure dispersion dudit composé 1 (et donc des particules solides 2 elles-mêmes) au sein d'une composition cosmétique et/ou dermatologique (comportant elle-même en particulier une phase huile ou assimilée).

Préférentiellement, ledit traitement de surface implique une greffe de molécules 5 à la surface respective desdites particules 2, comme illustré à la figure 2. Plus préférentiellement encore, lesdites molécules greffées 5 comprennent au moins des chaînes siliconées, des chaînes carbonées, des polymères naturels ou synthétiques, des molécules organiques, ou des oxydes de titane ou de silicium. Ainsi, avantageusement, ledit composé 1 comprend en particulier ladite matrice cireuse 3 et lesdites particules 2 traitées, lesquelles sont munies des et/ou comprennent lesdites molécules greffées 5. Lesdites particules 2 traitées sont de préférence dispersées de manière régulière au sein de la matrice cireuse 3, au moins en partie grâce auxdites molécules greffées 5, dont la présence renforce ainsi préférentiellement le caractère lipophile et/ou hydrophobe des particules 2 et/ou diminue avantageusement leur capacité d'absorption de matrice cireuse 3.

De façon avantageuse, ledit agent fonctionnel et ladite matrice cireuse 3 forment ensemble au moins 95%, de préférence au moins 97%, plus préférentiellement au moins 99%, en poids, plus préférentiellement encore la totalité, dudit composé 1. En effet, que ce soit pour réaliser ledit composé 1 ou pour disperser ce dernier au sein d'une composition cosmétique et / ou dermatologique, il n'est avantageusement pas nécessaire d'intégrer à ce dernier un autre composant que ladite matrice cireuse 3 et ledit agent fonctionnel, bien que cela reste éventuellement envisageable. Ainsi, de préférence, le composé 1 ne comprend pas de tensioactif, c'est-à-dire qu'il ne comprend en particulier aucune substance dont la fonction première est d'abaisser la tension superficielle, ou bien que le composé 1 comprend une telle substance mais en quantité non-utile, négligeable en poids relativement à la matrice cireuse 3 et / ou à l'agent fonctionnel.

L'invention concerne également en tant que tel, selon un deuxième aspect en partie illustré par un exemple de réalisation à la figure 3, un procédé de fabrication d'un composé 1, lequel est de préférence le composé 1 tel que décrit précédemment (et ci-après). Ainsi, le procédé de l'invention permet de fabriquer un composé 1 comprenant au moins 50% en poids d'un agent fonctionnel, de préférence tel que mentionné ci-avant, ledit composé 1 comprenant éventuellement (strictement) plus de 50%, de préférence (strictement) plus de 60%, plus préférentiellement au moins 70%, en poids, plus préférentiellement encore au moins 80%, en poids, voire 85%, en poids, dudit agent fonctionnel. La description qui précède concernant le composé 1 s'applique donc avantageusement également au procédé de fabrication selon l'invention (et inversement, la description qui suit concernant le procédé de fabrication s'applique donc également au composé 1 selon l'invention).

Selon l'invention, le procédé de fabrication comprend au moins une étape de chauffage C d'une matrice cireuse 3 afin d'obtenir une matrice fluide 3, ladite matrice cireuse 3 étant de préférence celle déjà mentionnée ci-avant. En d'autres termes, lors de ladite étape de chauffage Ç, ladite matrice cireuse 3 est chauffée, par exemple à l'aide d'au moins une résistance ou par convection, jusqu'à être fluidifiée, c'est-à-dire fondue ou suffisamment ramollie pour ne plus présenter la consistance d'un solide, formant ainsi une matrice cireuse fluide 3. Lors de ladite étape de chauffage, ladite matrice cireuse 3 est avantageusement chauffée à une température égale ou supérieure à son point de fusion et / ou de ramollissement. Ainsi, pendant l'étape de chauffage, ladite matrice cireuse 3 est par exemple chauffée à une température comprise entre 30 et 150 °C inclus, plus préférentiellement entre 45 et 130°C inclus, pour être fluidifiée, ou encore à une température comprise entre 55 et 95°C inclus ou entre 55 et 150°C inclus. De préférence, ladite matrice cireuse 3 à froid est ainsi sensiblement solide, tandis qu'à chaud, au-delà d'une certaine température, elle est sensiblement fluide. De préférence, l'expression « *matrice fluide* » désignent donc la matrice cireuse 3 chauffée jusqu'à fluidification.

Selon l'invention, le procédé comprend également une étape de traitement T (non illustrée) de particules solides 2 au cours de laquelle ces dernières subissent un traitement de surface entraînant une diminution de la capacité d'absorption de matrice fluide 3 par les particules solides 2 traitées, comparativement à des particules solides de même nature non traitées, lesdites particules solides 2 traitées formant ledit agent fonctionnel. Lesdites particules solides 2 sont de préférence telles que mentionnées ci-avant. En particulier, ladite étape de traitement T implique avantageusement, comme exposé ci-avant, une greffe de molécules 5 à la surface respective desdites particules 2 et / ou une augmentation du caractère lipophile et/ou hydrophobe des particules solides 2 traitées, comparativement à des particules solides de même nature non traitées.

Toujours selon l'invention, le procédé comprend en outre une étape de dispersion D dudit agent fonctionnel au sein de ladite matrice fluide 3. Ladite étape de dispersion D comprend avantageusement au moins une étape de mélange M desdites particules 2 traitées et de ladite matrice cireuse 3, cette dernière pouvant notamment être à froid, sous forme solide, ou à chaud, sous forme fluidifiée.

Selon l'invention, ledit composé 1 est solide à température ambiante et destiné à être dispersé par fluidification et/ou solubilisation au sein d'une composition cosmétique et / ou dermatologique pour y intégrer ledit agent fonctionnel, avantageusement comme décrit précédemment.

De façon avantageuse, le procédé comprend en outre une étape de broyage B desdites particules solides 2 visant à diminuer leur taille moyenne. Par exemple, à l'issue de ladite étape de broyage B, lesdites particules solides 2 présentent une taille moyenne sensiblement comprise entre 10 nm et 500 µm, de préférence entre 100 nm et 100 µm.

Préférentiellement, comme illustré à la figure 3, ladite phase de dispersion D comprend au moins en partie ladite étape de broyage B, de sorte que pendant au moins une partie de cette dernière, ladite matrice fluide 3 se trouve en mélange avec lesdites particules 2.

Selon un exemple de réalisation particulier de l'invention, comme illustré à la figure 3, le procédé comprend en outre une étape d'extrusion E dudit composé 1 pour que celui-ci se présente sous la forme de granulés, par exemple des pastilles ou des bâtonnets 4. De manière avantageuse, l'étape d'extrusion E est réalisée à chaud, ledit composé 1 présentant lors de cette étape une température comprise entre 60 et 80°C.

Avantageusement, le procédé comprend également, après l'étape de dispersion D, une étape de refroidissement F au cours de laquelle ledit composé 1 refroidit jusqu'à température ambiante de façon à former un solide.

Selon un exemple de réalisation donné à titre illustratif et non limitatif, illustré à la figure 3, le procédé implique successivement :
- ladite étape de mélange M, au cours de laquelle des particules solides 2 traitées et la matrice cireuse 3 sont mélangés à froid, la matrice 3 étant solide, à froid (température ambiante), sous forme de boulettes solides, tandis que les particules 2 se trouvent sous forme grossière, à une granulométrie trop importante et donc non acceptable, puis
- ladite étape de chauffage C, au cours de laquelle la matrice cireuse 3 est chauffée et donc fluidifiée, en même temps que les particules 2, qui restent solides, puis
- ladite étape de broyage B, au cours de laquelle les particules 2 sont broyées à une granulométrie acceptable, tout en étant/restant mélangées intimement avec la matrice fluide 3 qui subit elle-aussi, en quelque sorte, le broyage, puis
- une étape d'homogénéisation H, au cours de laquelle le mélange formé par les particules 2 broyées et la matrice fluide 3 est homogénéisé par tout moyen approprié (agitation, soufflerie, cisaillement...), puis
- ladite étape d'extrusion E, au cours de laquelle le mélange chaud est compressé afin de traverser une filière 6 pour obtenir un produit long en forme de cylindre, puis
- ladite étape de refroidissement F, au cours de laquelle le produit long est progressivement refroidi, puis
- une étape de découpe S, au cours de laquelle le produit long est découpé, séparé en plusieurs solides distincts les uns des autres, de préférence de type granulés, par exemple des pastilles ou des bâtonnets 4,
ladite étape de dispersion D comprenant ici les étapes de mélange M, chauffage C, broyage B et homogénéisation H.

Le procédé, en particulier selon cet exemple de réalisation, implique avantageusement l'utilisation d'une extrudeuse à bi-vis chauffante 7, au sein de laquelle les étapes exposées ci-dessus sont réalisées.

Il est particulièrement avantageux de réaliser ladite étape de broyage B de manière à ce que la granulométrie (moyenne) desdites particules solides 2 soit diminuée, et à ce que lesdites particules solides 2 en cours de broyage soient en mélange avec la matrice fluide 3, laquelle subit donc également, en quelque sorte, ledit broyage. En d'autres termes, lors de ladite étape de broyage B les particules 2 sont broyées en présence de la matrice fluide 3. Cette étape de broyage B peut être ainsi concomitante avec ladite étape de mélange M. L'étape de broyage B est dans ce cas particulièrement simple et aisée à réaliser, puisqu'il suffit de broyer la matrice cireuse 3 en mélange avec les particules solides 2. Grâce à l'étape de broyage B, l'éventuelle étape d'extrusion E (qui est réalisée de préférence après) est grandement facilitée. Cette étape de broyage B peut en outre avantageusement permettre de se passer d'un broyage fin des particules solides 2 avant leur mélange avec la matrice cireuse 3, ce qui évite de devoir gérer des particules solides 2 très fines, présentant donc un fort caractère pulvérulent, avec les inconvénients en termes de stockage, de sécurité et de mise en oeuvre qui en découlent. Des tests comparatifs ont en outre démontré que l'étape de broyage B conférait à des exemples de composés 1 de l'invention une excellente dispersibilité en phase grasse (d'une composition cosmétique par exemple), en comparaison de composés équivalents mais n'ayant pas été réalisés par un procédé incluant ladite étape de broyage B et présentant donc des particules solides 2 dont la granulométrie reste sensiblement la même pendant tout le processus de fabrication.

L'invention concerne également en tant que tel, selon un troisième aspect, un procédé de fabrication d'une composition cosmétique et/ou dermatologique, comprenant une étape de redispersion R au cours de laquelle un composé 1, tel que décrit précédemment, est ajouté à puis mélangé avec un ou plusieurs composants acceptables en cosmétique et/ou en dermatologie. La description qui précède concernant le composé 1 et le procédé de fabrication d'un composé 1 s'applique avantageusement également au procédé de fabrication d'une composition selon l'invention.

Selon une première variante, ladite étape de redispersion R comprend en outre une étape de solubilisation dudit composé 1 par un ou plusieurs desdits composants acceptables en cosmétique et / ou en dermatologie. De manière avantageuse, ladite étape de solubilisation comprend au moins la solubilisation (c'est-à-dire notamment la dispersion) du composé 1 au sein d'une phase grasse (en particulier huile) ou d'une portion de ladite phase grasse de ladite composition cosmétique et/ou dermatologique.

Selon une seconde variante, compatible avec la première décrite ci-avant, ladite étape de redispersion R comprend en outre une étape de fluidification, dans laquelle ledit composé 1 ajouté est chauffé au moins jusqu'à fluidification. Ladite étape de redispersion R peut également comprendre ladite étape de solubilisation et ladite étape de fluidification, par exemple l'une après l'autre ou selon un autre exemple de manière concomitante.

De préférence, pour l'étape de redispersion R et notamment pour l'étape de solubilisation et / ou pour l'étape de fluidification, un ou plusieurs desdits composants acceptables en cosmétique et/ou en dermatologie sont sensiblement fluides à température ambiante, facilitant la redispersion dudit composé 1 que ce soit via sa solubilisation et/ou via sa fluidification.

Afin de mettre en évidence l'amélioration de la teneur en particules solides 2 au sein du composé 1, différents exemples de composés 1, désignés A1 à A3, conformes à l'invention, c'est-à-dire comprenant des particules solides 2 ayant subi un traitement de surface entraînant une diminution de la capacité d'absorption de matrice cireuse, ont été réalisés, à titre illustratif et non limitatif uniquement. Des exemples de composés désignés A4 à A6 ont également été réalisés, mais de manière non conforme à l'invention, c'est-à-dire qu'ils comprennent ici des particules solides de même nature que celle des particules solides des exemples de composés A1 à A3, mais sans traitement de surface.

Les exemples de composés A1 à A6 ont été réalisés selon le tableau 1, en ce qui concerne leurs particules solides respectives (ici des pigments).

**Tableau 1**

| Composés | Particules solides (pigments) | Nom commercial, INCI, CAS et/ou EINEC des pigments | Traitement de surface des particules solides (pigments) | Composé conforme à l'invention ? |
|---|---|---|---|---|
| A1 | dioxyde de titane traité (pigments blancs) | MiyoNAT VAA-White | oui | oui |
| A2 | oxyde de fer rouge traité | MiyoNAT VAA-Red | oui | oui |
| A3 | oxyde de fer noir traité | MiyoNAT VAA-Black | oui | oui |
| A4 | dioxyde de titane (pigments blancs) | CI 77891 | aucun | non |
| | | 13463-67-7 | | |
| | | 236-675-5 | | |
| A5 | oxyde de fer rouge | CI 77491 | aucun | non |
| | | 1309-37-1 | | |
| | | 215-168-2 | | |
| A6 | oxyde de fer noir | CI 77499 | aucun | non |
| | | 1317-61-9 | | |
| | | 215-277-5 | | |

Les particules solides des composés A4 à A6 sont dans ces exemples des pigments non traités, dont les appellations INCI, CAS et EINEC sont données dans le tableau 1. Les particules solides 2 des composés A1 à A3 sont dans ces exemples non limitatifs des pigments traités, c'est-à-dire qui particules solides 2 (de type pigment) ayant subi un traitement de surface entraînant une diminution de la capacité d'absorption de matrice cireuse. Les noms commerciaux respectifs (à la date dépôt de la présente demande de brevet) de ces pigments traités sont donnés au tableau 1. Les pigments traités des composés A1 à A3 sont commercialisés sous leurs noms commerciaux respectifs notamment par la société Miyoshi.

Chacun de ces exemples de composés A1 à A6 a été préparé en plusieurs formulations respectives, chacune contenant l'une des proportions respectives suivantes, en poids, de particules solides/matrice cireuse : 40/60, 50/50, 60/40, 70/30, 80/20, et 85/15. Ainsi, 36 formulations différentes de ces exemples de composés ont été préparées, dont 18 sont des formulations des exemples de composés 1 conformes à l'invention (A1 à 14), et 18 ne le sont pas (A4 à A6). Bien évidemment, la matrice cireuse 3 mise en oeuvre est la même pour tous les exemples de composés A1 à A6, et par exemple formée de polyméthylsilsesquioxane. L'exemple de composé A1 désigne par exemple toutes les formulations de cet exemple de composé, à savoir six formulations avec différentes proportions respectives (mentionnées ci-avant), en poids, de particules solides/matrice cireuse.

Le traitement de surface des pigments des exemples de composés A1 à A3 peut par exemple être réalisé ici avec un traitement à base d'acides aminés, de préférence conformément à l'enseignement du brevet US-4,606,914, les acides aminés se présentant en particulier sous la forme de sels métalliques d'acide N-acylaminé. Ainsi, ledit traitement de surface consiste par exemple à enduire des particules solides (et notamment des pigments) brutes, c'est-à-dire non-traitées, avec, en poids de particules solides brutes, 0,5 à 10% d'un ou plusieurs sels métalliques d'acide(s) N-acylaminé(s). L'acide N-acylaminé est par exemple un acide N-acyl-L-glutamique, tandis que le métal est par exemple l'aluminium, le magnésium, le calcium, le zinc ou encore le titane. Le sel métallique d'acide N-acylaminé est par exemple réalisé à l'aide de myristoyl glutamate de sodium et d'hydroxyde d'aluminium. Un tel traitement de surface peut en particulier être utilisé pour les exemples présentés ici, à titre illustratif et non limitatif, ainsi que dans le reste de la description ci-avant et ci-après. Ledit traitement de surface, en particulier ledit traitement à l'aide de sels métalliques d'acide N-acylaminé, augmente de préférence le caractère hydrophobe et la dispersibilité dans l'huile des particules solides 2 traitées par rapport aux particules solides brutes (non traitées).

Les exemples de composés A1 à A6 ont été réalisés via le procédé de l'invention, incluant ici au moins les étapes de chauffage C, de dispersion D et de broyage B déjà mentionnées. Ces exemples de composés A1 à A6 ont en outre subi ladite étape d'extrusion E, et se trouvent tous sous forme de granulés solides à température ambiante.

Les résultats des formulations des exemples de composés sont donnés dans le tableau 2. La mention « *oui* » désigne la réussite d'une formulation de composé homogène, c'est-à-dire en d'autres termes une dispersion solide homogène de pigments et de matrice cireuse, de préférence facilement extrudable, et la mention « *non* » désigne l'échec d'une formulation de composé, la formulation obtenue étant alors généralement hétérogène voire de nature partiellement pulvérulente, résultat d'une mauvaise répartition des pigments et de la matrice cireuse. En règle générale, plus la teneur en pigments (c'est-à-dire en particules solides) est importante, plus il est difficile de réaliser une formulation de composé réussie.

**Tableau 2**

| Ratio pigments / matrice (en poids) | 40/60 | 50/50 | 60/40 | 70/30 | 80/20 | 85/15 |
|---|---|---|---|---|---|---|
| Composés | | | | | | |
| A1 | oui | oui | oui | oui | oui | oui |
| A2 | oui | oui | oui | oui | oui | oui |
| A3 | oui | oui | oui | oui | oui | oui |
| A4 | oui | oui | non | non | non | non |
| A5 | oui | oui | non | non | non | non |
| A6 | oui | oui | non | non | non | non |

Ainsi, avec les préparations de dispersion solide de pigments traités A1 à A3, il a été possible d'obtenir des formulations solides homogènes contenant toutes les teneurs de pigments testées, c'est-à-dire ici 40 à 85% en poids de pigments traités.

En revanche, avec les formulations des exemples de composés A4 à A6, il a été seulement possible d'obtenir un résultat acceptable pour des teneurs en pigments relativement basses (40 et 50% en poids de pigments non traités). Au-delà de 50% en poids de pigment non traités, il n'a pas été possible d'obtenir des formulations de composés solides homogènes satisfaisants, mais uniquement des mélanges poudreux et hétérogènes de pigments et de matrice cireuse, qui présentent une mauvaise dispersibilité et peuvent en outre compliquer voire empêcher la réalisation de ladite étape d'extrusion E.

D'après les résultats précités, il a été démontré que le traitement de surface des particules solides 2 (des pigments dans ces exemples particuliers), dans les exemples de composés 1 de la présente invention, permettait d'augmenter significativement la concentration desdites particules solides pigments dans la matrice cireuse 3.

Afin de mettre en évidence l'excellente (re)dispersibilité du composé 1, différents exemples de composés 1, désignés A7 à A12, conformes à l'invention, ont été réalisés, à titre illustratif et non limitatif uniquement, et sont présentés dans le tableau 3.

**Tableau 3**

| Composé | Particules solides (pigments) - Nom commercial | Matrice cireuse | Ratio pigments/ matrice (en poids) |
|---|---|---|---|
| A7 | dioxyde de titane traité (MiyoNAT VAA-White) | cire | 85/15 |
| A8 | oxyde de fer rouge traité (MiyoNAT VAA-Red) | cire | 85/15 |
| A9 | oxyde de fer noir traité (MiyoNAT VAA-Black) | cire | 85/15 |
| A10 | dioxyde de titane traité (pigments blancs) (MiyoNAT VAA-White) | résine de silicone | 85/15 |
| A11 | oxyde de fer rouge traité (MiyoNAT VAA-Red) | résine de silicone | 85/15 |
| A12 | oxyde de fer noir traité (MiyoNAT VAA-Black) | résine de silicone | 85/15 |

Les pigments traités (qui sont donc des particules solides ayant subi un traitement de surface entraînant une diminution de la capacité d'absorption de matrice cireuse) des composés A7 à A12 présentés au tableau 3 sont les mêmes que ceux des composés A1 à A3. Le terme « *cire* » des composés A7 à A9 dans le tableau 3 désigne par exemple une cire d'ester d'huile d'olive, tandis que la résine de silicone des composés A10 à A12 dans le tableau 3 est par exemple formée par le polyméthylsilsesquioxane.

Les exemples de composés A7 à A12 ont été réalisés via le procédé de l'invention, incluant ici au moins les étapes de chauffage C, de dispersion D et de broyage B déjà mentionnées. Ces exemples de composés A7 à A12 ont en outre subi ladite étape d'extrusion E, et se trouvent tous sous forme de granulés solides à température ambiante. Les particules solides 2 (ici différents pigments) de ces exemples de composés A7 à A12, ont subi un traitement de surface conformément à l'invention, par exemple à l'aide d'un ou plusieurs sels métalliques d'acide(s) N-acylaminé(s), de préférence conformément à l'enseignement du brevet US-4,606,914.

La (re)dispersibilité des exemples de composés A7 à A12 a été évaluée en mélangeant chacun d'entre eux, séparément, dans différents types de corps gras liquides (huile apolaire de type alcane linéaire ou branché, ou huile polaire de type ester linéaire ou branché, ou huile polaire de type triglycéride, ou huile silicone linéaire de type diméthicone, ou huile silicone cyclique de type cyclopentasiloxane, ou huile silicone branchée de type méthyl triméthicone), suivant un ratio, en poids, d'une partie de dispersion solide de pigment dans deux parties de corps gras liquide. Chacun de ces corps gras liquides peut ainsi être destiné à former une phase grasse ou une portion de phase grasse d'une composition cosmétique et/ou dermatologique. En d'autres termes, l'évaluation de la dispersibilité des exemples de composés A7 à A12 dans ces différents corps gras permet d'évaluer la faculté / la facilité de dispersion homogène des exemples de composés A7 à A12 au sein d'une composition cosmétique et/ou dermatologique respective.

La dispersibilité des exemples de composés A7 à A12 dans un corps gras liquide respectif a été réalisée au moyen d'équipements de mélange relativement courant (de type défloculeuse, à 3300 rpm de 5 à 15 min, ou de type disperseur ultra-turrae, à 8000 rpm, de 5 à 15 min). Aucune étape de broyage fin de pigments seuls, de type tricylindre par exemple, n'a été effectuée préalablement à la réalisation des exemples de composés A7 à A12.

Les exemples de composés A7 à A9 ont été mélangées aux corps gras liquides à chaud (entre 60 et 80°C) pour former des mélanges respectifs M7 à M9. Les exemples de composés A10 à A12 ont été mélangées aux corps gras liquides à température ambiante (25°C) pour former des mélanges respectifs M10 à M12.

La dispersibilité (c'est-à-dire l'homogénéité de répartition) des exemples de composés A7 à A12 au sein de leurs corps gras liquides respectifs a été évaluée par observation des mélanges M7 à M12 au microscope optique, étalement entre deux plaques de verre et étalement sur jauge de finesse de broyage (dite aussi jauge Hegman) à 100 µm et 25 µm, et comparaison avec des compositions M7' à M12' incluant des pigments et corps gras liquides de même nature (mais non-traités en ce qui concerne les pigments) et en mêmes proportions que les mélanges A7 à A12. Les pigments des compositions M7' à M12' ont cependant été réalisés avec des équipements de broyage fin (broyage par tricylindre par exemple) et dispersés directement sous forme pulvérulente au sein de leur corps gras liquides respectifs pour former lesdites compositions M7' à M12', sans dispersion intermédiaire dans une matrice cireuse.

Les résultats des observations décrites ci-avant montrent que les mélanges M7 à M12, dont les pigments avaient subi un traitement de surface puis été intégrés au sein des exemples de composés A7 à A12 (eux-mêmes intégrés au sein des mélanges M7 à M12), présentaient la même finesse de broyage (taille de particules, homogénéité de la dispersion) que les mélanges M7' à M12', dont les pigments avaient subi un broyage fin, au tricylindre par exemple, mais pas de traitement de surface ni d'intégration au sein d'un composé 1 de l'invention destiné à être redispersé.

D'après les résultats précités, il a été démontré que les particules solides 2 (ici des pigments) présents dans les exemples de composés A7 à A12 solides conformes à la présente invention s'étaient dispersés uniformément dans les corps gras liquide (considérés comme des portions de composition cosmétique et/ou dermatologique) à une forte teneur (en poids) en particules solides 2, et ce en une période de temps limitée (durée normale de mélange). En conséquence, le composé 1 de la présente invention permet d'obtenir une excellente facilité de redispersion de particules solides 2, et notamment de pigments, au sein d'une composition cosmétique et/ou dermatologique, et en particulier au sein d'une phase grasse (huile) d'une composition cosmétique et/ou dermatologique.

Des exemples de compositions cosmétiques et/ou dermatologiques, réalisés selon le procédé de fabrication de composition de l'invention, intégrant chacun au moins un composé 1 conforme à l'invention, sont décrites ci-après, de manière illustrative et non limitative :
- Composition M20 : fond de teint réalisé par émulsion eau-dans-silicone, ayant la composition suivante (en poids) :
   > Phase grasse
      - Tensio-actif de type PEG-10 diméthicone ou de type PEG-12 diméthicone ou de type lauryl PEG-9 polydiméthylsiloxyethyl diméthicone : 3,00%
      - Dispersion de disteardimonium hectorite et de propylène carbonate : 1,00%
      - Cyclopentasiloxane : 17,25%
      - Huile apolaire de type alcane ou huile polaire de type ester ou huile polaire de type triglycéride ou huile silicone linéaire de type diméthicone ou huile silicone cyclique de type cyclopentasiloxane : 10,00%
      - Composé 1 conforme à l'invention, choisi parmi les exemples de composés A7 à A12 : 10,00%
   > Phase aqueuse
      - Sulfate de magnésium : 0,75%
      - Gomme xanthane : 0,15%
      - Butylène glycol : 5,00%
      - Conservateur : 0,75%
      - Eau déminéralisée : q.s.
- Composition M21 : émulsion eau-dans-huile, ayant la composition suivante (en poids) :
   > Phase grasse
      - Tensio-actif de type polyglycéryl-3 diisostéarate : 3,00%
      - Cires : 2,00%
      - Glycéryl béhénate : 1,00%
      - Huile isononyl isononanoate : 10,00%
      - Huile apolaire de type alcane ou huile polaire de type ester ou huile polaire de type triglycéride ou huile silicone linéaire de type diméthicone ou huile silicone cyclique de type cyclopentasiloxane : 5,00%
      - Composé 1 conforme à l'invention, choisi parmi les exemples de composés A7 à A12 : 10,00%
   > Phase aqueuse
      - Chlorure de sodium : 1,50%
      - Sulfate de magnésium : 1,50%
      - Gomme xanthane : 0,20%
      - Butylène glycol : 3,00%
      - Conservateur : 0,30%
      - Eau déminéralisée : q.s.
- Composition M22 : fond de teint réalisé par émulsion eau-dans-huile, à froid, ayant la composition suivante (en poids) :
   > Phase grasse
      - Tensio-actif de type polyglycéryl-3 polyricinoléate : 8,00%
      - Dispersion de disteardimonium hectorite et de propylène carbonate : 5,00%
      - Huile de type alcane : 13,70%
      - Agent texturant de type silice amorphe : 5,00%
      - Conservateur : 1,00%
      - Composé 1 conforme à l'invention, choisi parmi les exemples de composés A10 à A12 : 10,00%
   > Phase aqueuse
      - Chlorure de Sodium : 1,00%
      - Glycérine : 3,00%
      - Gomme xanthane : 0,30%
      - Eau déminéralisée : q.s.
- Composition M23 : fond de teint anhydre en stick, ayant la composition suivante (en poids) :
   > Phase grasse
      - Cires : 70,00%
      - Huile apolaire de type alcane ou huile polaire de type ester ou huile polaire de type triglycéride ou huile silicone de type diméthicone ou huile silicone de type cyclopentasiloxane : 20,00%
      - Composé 1 conforme à l'invention, choisi parmi les exemples de composés A7 à A9 : 10,00%.
- Composition M24 : rouge à lèvre, ayant la composition suivante (en poids) :
   > Phase grasse
      - Cires : 70,00%
      - Huile apolaire de type alcane ou huile polaire de type ester ou huile polaire de type triglycéride ou huile silicone de type diméthicone ou huile silicone de type cyclopentasiloxane : 14.50%
      - Agent texturant de type Silice amorphe : 5,00%
      - Tocophérols : 0,50%
      - Composé 1 conforme à l'invention, choisi parmi les exemples de composés A7 à A9 : 10.00%.

Pour chacun des exemples de compositions M20 à M24, le composé 1 a été mélangé avec la phase grasse, soit à température ambiante (25°C environ) pour les exemples de composés A10 à A12, soit à chaud (60-80°C ou 75-85°C) pour les exemples de composés A7 à A9. Chaque composé 1 est dans ces exemples de réalisation de compositions toujours mélangé en premier lieu à la phase grasse respective, à l'aide d'une défloculeuse et/ou d'un rotor-stator.

La stabilité des exemples de compositions M20 à M22 (qui sont des émulsions) a été suivie pendant deux mois, à température ambiante (25°C), à 40°C et à 50°C. Leurs viscosités respectives ont également été suivies pendant deux mois à température ambiante (25°C).

Aucun changement significatif n'a été observé. Le composé 1 de l'invention permet donc non seulement une excellente redispersibilité des particules solides 2 au sein d'une composition cosmétique et/ou dermatologique, mais de surcroît n'impacte pas négativement cette dernière quant à sa stabilité dans le temps.

L'homogénéité des exemples de compositions M20 à M24 a été évaluée par observation de ces derniers au microscope optique, étalement entre deux plaques de verre et étalement sur jauge de finesse de broyage (dite aussi jauge Hegman) à 100 µm et 25 µm, et comparaison avec des exemples de compositions M20' à M24' incluant des particules solides 2 (pigments des exemples de composés A7 à A12) et autres composants de même nature (mais non-traités en ce qui concerne les particules solides 2) et en mêmes proportions que les exemples de compositions M20 à M24. Les pigments des exemples de compositions M20' à M24' ont été réalisés avec des équipements de broyage fin (broyage par tricylindre par exemple) dispersés sous forme pulvérulente au sein de leur corps gras liquides respectifs pour former lesdits exemples de compositions M20' à M24', sans dispersion intermédiaire dans une matrice cireuse.

Les résultats des observations décrites ci-avant montrent que les exemples de compositions M20 à M24, préparés à partir des exemples de composés A7 à A12 de l'invention, présentaient la même finesse de broyage (taille de particules, homogénéité de la dispersion) que les exemples de compositions M20' à M24', dont les pigments avaient subi un broyage fin, au tricylindre par exemple, mais pas de traitement de surface ni d'intégration au sein d'un composé 1 de l'invention destiné à être redispersé. En d'autres termes, les exemples de compositions cosmétiques M20 à M24, contenant chacun un composé 1 respectif selon l'invention incluant lui-même des particules solides 2, présentaient chacun la même finesse d'émulsion (taille de particules, homogénéité de la dispersion) que si leurs particules solides avaient été broyés finement et longuement dans un tricylindre, sans passage par un composé 1 de l'invention ni traitement de surface, et introduite directement sous forme pulvérulente au sein des exemples de compositions.

### POSSIBILITE D'APPLICATION INDUSTRIELLE

L'invention permet ainsi d'obtenir un composé 1 comportant une importante teneur en agent fonctionnel et facilitant le transport, le stockage et la manipulation de ce dernier.

## Revendications

1. Composé (1) comprenant au moins 50% en poids d'un agent fonctionnel, **caractérisé en ce qu'**il comprend également une matrice cireuse (3) au sein de laquelle ledit agent fonctionnel est dispersé, ledit composé (1) étant solide à température ambiante et destiné à être dispersé par fluidification et / ou solubilisation au sein d'une composition cosmétique et / ou dermatologique pour y intégrer ledit agent fonctionnel, ledit agent fonctionnel étant formé de particules solides (2) ayant subi un traitement de surface entraînant une diminution de la capacité d'absorption de matrice cireuse (3) par lesdites particules solides (2) traitées, comparativement à des particules solides de même nature non traitées.

2. Composé (1) solide selon la revendication précédente, **caractérisé en ce que** ledit traitement de surface permet également d'augmenter le caractère lipophile et/ou hydrophobe des particules solides (2) traitées, comparativement à des particules solides de même nature non traitées.

3. Composé (1) solide selon la revendication 1 ou 2, **caractérisé en ce que** ledit traitement de surface implique une greffe de molécules (5) à la surface respective desdites particules (2), lesdites molécules greffées (5) comprenant de préférence au moins des chaînes siliconées, des chaînes carbonées, des polymères naturels ou synthétiques, des molécules organiques, ou des oxydes de titane ou de silicium.

4. Composé (1) selon l'une des revendications précédentes, **caractérisé en ce que** ladite matrice cireuse (3) comprend au moins une cire naturelle, minérale et / ou synthétique, un corps gras solide à température ambiante issu du fractionnement d'une ou plusieurs huile(s) naturelle(s) et/ou synthétique(s), une résine naturelle et/ou synthétique, une cire de silicone ou de polymère de silicone, ou bien un mélange de ceux-ci.

5. Composé (1) selon l'une des revendications précédentes, **caractérisé en ce que** ladite matrice cireuse (3) comprend du polyméthylsilsesquioxane, et est par exemple principalement formée par ce dernier.

6. Composé (1) selon l'une des revendications précédentes, **caractérisé en ce que** lesdites particules solides (2) présentent une taille moyenne comprise entre 10 nm et 500 µm, de préférence entre 100 nm et 100 µm.

7. Composé (1) selon l'une des revendications précédentes, **caractérisé en ce qu'**il se présente sous la forme de granulés, par exemple des pastilles ou des bâtonnets (4), obtenus par extrusion.

8. Composé (1) selon l'une des revendications précédentes, **caractérisé en ce que** ledit agent fonctionnel forme au moins 70%, de préférence au moins 80%, en poids, dudit composé (1).

9. Composé (1) selon l'une des revendications précédentes, caractérisé en ce ledit agent fonctionnel et ladite matrice cireuse (3) forment ensemble au moins 95%, de préférence au moins 97%, plus préférentiellement au moins 99%, en poids, plus préférentiellement encore la totalité, dudit composé (1).

10. Composé (1) selon l'une des revendications précédentes, **caractérisé en ce qu'**il ne comprend pas de tensioactif.

11. Procédé de fabrication d'un composé (1) comprenant au moins 50% en poids d'un agent fonctionnel, **caractérisé en ce qu'**il comprend au moins :
- une étape de chauffage (C) d'une matrice cireuse (3) afin d'obtenir une matrice fluide (3),
- une étape de traitement (T) de particules solides (2) au cours de laquelle ces dernières subissent un traitement de surface entraînant une diminution de la capacité d'absorption de matrice fluide (3) par les particules solides (2) traitées, comparativement à des particules solides (2) de même nature non traitées, lesdites particules solides (2) traitées formant ledit agent fonctionnel,
- une étape de dispersion (D) dudit agent fonctionnel au sein de ladite matrice fluide (3),
ledit composé (1) étant solide à température ambiante et destiné à être dispersé par fluidification et / ou solubilisation au sein d'une composition cosmétique et / ou dermatologique pour y intégrer ledit agent fonctionnel.

12. Procédé selon la revendication précédente, **caractérisé en ce qu'**il comprend en outre une étape de broyage (B) desdites particules solides (2) visant à diminuer leur taille moyenne, et **en ce qu'**à l'issue de ladite étape de broyage (B), lesdites particules solides (2) présentent de préférence une taille moyenne sensiblement comprise entre 10 nm et 500 µm, plus préférentiellement entre 100 nm et 100 µm.

13. Procédé selon la revendication 11 ou 12, **caractérisé en ce qu'**il comprend une étape d'extrusion (E) dudit composé (1) pour que celui-ci se présente sous la forme de granulés, par exemple des pastilles ou des bâtonnets (4), l'étape d'extrusion (E) étant de préférence réalisée à chaud, ledit composé (1) présentant lors de cette étape une température avantageusement comprise entre 60 et 80°C.

14. Procédé de fabrication d'une composition cosmétique et / ou dermatologique comprenant une étape de redispersion (R) au cours de laquelle un composé (1) selon l'une quelconque des revendications 1 à 10 est ajouté à puis mélangé avec un ou plusieurs composants acceptables en cosmétique et / ou en dermatologie.

15. Procédé de fabrication d'une composition cosmétique et / ou dermatologique selon la revendication précédente, **caractérisé en ce que** ladite étape de redispersion (R) comprend en outre une étape de solubilisation dudit composé (1) par un ou plusieurs desdits composants acceptables en cosmétique et / ou en dermatologie, et/ou **en ce que** ladite étape de redispersion (R) comprend en outre une étape de fluidification, dans laquelle ledit composé (1) ajouté est chauffé au moins jusqu'à fluidification.

## Patentansprüche

1. Verbindung (1), umfassend mindestens 50 Gew.-% eines funktionellen Mittels, **dadurch gekennzeichnet, dass** sie auch eine wachsartige Matrix (3) umfasst, im Inneren derer das genannte funktionelle Mittel dispergiert ist, wobei die genannte Verbindung (1) bei Umgebungstemperatur fest ist und dazu bestimmt ist, durch Fluidisierung und/oder Solubilisierung im Inneren einer kosmetischen und/oder dermatologischen Zusammensetzung dispergiert zu werden, um das funktionelle Mittel darin zu integrieren, wobei das genannte funktionelle Mittel aus festen Partikeln (2) gebildet ist, welche einer Oberflächenbehandlung unterzogen wurden, die eine Verringerung der Absorptionskapazität der wachsartigen Matrix (3) durch die genannten behandelten festen Partikel (2) bewirkt, verglichen mit unbehandelten festen Partikeln derselben Beschaffenheit.

2. Feste Verbindung (1) nach dem vorhergehenden Anspruch, **dadurch gekennzeichnet, dass** es die genannte Oberflächenbehandlung auch gestattet, den lipophilen und/oder hydrophoben Charakter der behandelten festen Partikel (2) zu erhöhen, verglichen mit unbehandelten festen Partikeln derselben Beschaffenheit.

3. Feste Verbindung (1) nach Anspruch 1 oder 2, **dadurch gekennzeichnet, dass** die genannte Oberflächenbehandlung ein Pfropfen von Molekülen (5) auf die jeweilige Oberfläche der genannten Partikel (2) impliziert, wobei die genannten aufgepfropften Moleküle (5) vorzugsweise mindestens silikonisierte Ketten, karbonisierte Ketten, natürliche oder synthetische Polymere, organische Moleküle oder Titan- oder Siliziumoxide umfassen.

4. Verbindung (1) nach einem der vorhergehenden Ansprüche, **dadurch gekennzeichnet, dass** die genannte wachsartige Matrix (3) mindestens ein natürliches, mineralisches und/oder synthetisches Wachs, einen bei Umgebungstemperatur festen Fettkörper, der aus der Fraktionierung eines oder mehrerer natürlicher und/oder synthetischer Öle erhalten wird, ein natürliches und/oder synthetisches Harz, ein Silikon- oder Silikonpolymerwachs oder auch eine Mischung davon umfasst.

5. Verbindung (1) nach einem der vorhergehenden Ansprüche, **dadurch gekennzeichnet, dass** die genannte wachsartige Matrix (3) Polymethylsilsesquioxan umfasst und zum Beispiel hauptsächlich aus diesem Letzteren gebildet ist.

6. Verbindung (1) nach einem der vorhergehenden Ansprüche, **dadurch gekennzeichnet, dass** die genannten festen Partikel (2) eine mittlere Größe zwischen 10 nm und 500 µm, vorzugsweise zwischen 100 nm und 100 µm, aufweisen.

7. Verbindung (1) nach einem der vorhergehenden Ansprüche, **dadurch gekennzeichnet, dass** sie sich in der Form von Granulaten, zum Beispiel Pastillen oder Stiften (4) präsentiert, die durch Extrusion erhalten werden.

8. Verbindung (1) nach einem der vorhergehenden Ansprüche, **dadurch gekennzeichnet, dass** das genannte funktionelle Mittel mindestens 70 Gew.-%, vorzugsweise mindestens 80 Gew.-% der genannten Verbindung (1) bildet.

9. Verbindung (1) nach einem der vorhergehenden Ansprüche, **dadurch gekennzeichnet, dass** das genannte funktionelle Mittel und die genannte wachsartige Matrix (3) gemeinsam mindestens 95 Gew.-%, vorzugsweise mindestens 97 Gew.-%, mehr bevorzugt mindestens 99 Gew.-%, noch mehr bevorzugt die Gesamtheit der genannten Verbindung (1) bilden.

10. Verbindung (1) nach einem der vorhergehenden Ansprüche, **dadurch gekennzeichnet, dass** sie keinen grenzflächenaktiven Stoff umfasst.

11. Verfahren zur Herstellung einer Verbindung (1), umfassend mindestens 50 Gew.-% eines funktionellen Mittels, **dadurch gekennzeichnet, dass** es mindestens umfasst:
- einen Schritt (C) zum Erhitzen einer wachsartigen Matrix (3), um eine fluide Matrix (3) zu erhalten,
- einen Schritt (T) zum Behandeln von festen Partikeln (2), in dem diese Letzteren einer Oberflächenbehandlung unterzogen werden, die eine Verringerung der Absorptionskapazität der wachsartigen Matrix (3) durch die behandelten festen Partikel (2) bewirkt, verglichen mit unbehandelten festen Partikeln (2) derselben Beschaffenheit, wobei die genannten behandelten festen Partikel (2) das genannte funktionelle Mittel bilden,
- einen Schritt (D) zum Dispergieren des genannten funktionellen Mittels im Inneren der genannten fluiden Matrix (3),
wobei die genannte Verbindung (1) bei Umgebungstemperatur fest ist und dazu bestimmt ist, durch Fluidisierung und/oder Solubilisierung im Inneren einer kosmetischen und/oder dermatologischen Zusammensetzung dispergiert zu werden, um das funktionelle Mittel darin zu integrieren.

12. Verfahren nach dem vorhergehenden Anspruch, **dadurch gekennzeichnet, dass** es außerdem einen Schritt (B) zum Zerkleinern der genannten festen Partikel (2) umfasst, der dazu dient, ihre mittlere Größe zu verringern, und dadurch, dass am Ende des genannten Zerkleinerungsschritts (B) die genannten festen Partikel (2) vorteilhaft eine mittlere Größe aufweisen, die im Wesentlichen zwischen 10 nm und 500 µm, mehr bevorzugt zwischen 100 nm und 100 µm, liegt.

13. Verfahren nach Anspruch 11 oder 12, **dadurch gekennzeichnet, dass** es einen Schritt (E) zum Extrudieren der genannten Verbindung (1) umfasst, damit sich diese in der Form von Granulaten, zum Beispiel Pastillen oder Stiften (4), präsentiert, wobei der Extrusionsschritt (E) vorzugsweise heiß durchgeführt wird, wobei die genannte Verbindung (1) während dieses Schritts eine Temperatur vorteilhaft zwischen 60 und 80 °C aufweist.

14. Verfahren zur Herstellung einer kosmetischen und/oder dermatologischen Zusammensetzung, umfassend einen Redispergierschritt (R), in dem eine Verbindung (1) nach einem der Ansprüche 1 bis 10 zugegeben wird und dann mit einer oder mehreren anderen kosmetisch und/oder dermatologisch annehmbaren Komponenten gemischt wird.

15. Verfahren zur Herstellung einer kosmetischen und/oder dermatologischen Zusammensetzung nach dem vorhergehenden Anspruch, **dadurch gekennzeichnet, dass** der genannte Redispergierschritt (R) außerdem einen Solubilisierungsschritt der genannten Verbindung (1) durch eine oder mehrere der genannten anderen kosmetisch und/oder dermatologisch annehmbaren Komponenten umfasst, und/oder dadurch, dass der genannte Redispergierschritt (R) außerdem einen Fluidisierungsschritt umfasst, in dem die genannte zugegebene Verbindung (1) mindestens bis zur Fluidisierung erhitzt wird.

## Claims

1. A compound (1) comprising at least 50 weight% of a functional agent, **characterized in that** it also comprises a waxy matrix (3) within which said functional agent is dispersed, said compound (1) being solid at room temperature and intended to be dispersed by fluidification and/or solubilization within a cosmetic and/or dermatological composition to integrate said functional agent therein, said functional agent being formed by solid particles (2) having undergone a surface treatment causing a reduction of the capacity of absorption of the waxy matrix (3) by said treated solid particles (2), in comparison with non-treated solid particles of the same kind.

2. The solid compound (1) according to the preceding claim, **characterized in that** said surface treatment also allows increasing the lipophilic and/or hydrophobic nature of the treated solid particles (2), in comparison with non-treated solid particles of the same kind.

3. The solid compound (1) according to claim 1 or 2, **characterized in that** said surface treatment involves grafting of molecules (5) at the respective surface of said particles (2), said grafted molecules (5) comprising preferably at least silicone chains, carbonated chains, natural or synthetic polymers, organic molecules, or titanium or silicon oxides.

4. The compound (1) according to any of the preceding claims, **characterized in that** said waxy matrix (3) comprises at least one natural, mineral and/or synthetic wax, a fatty body solid at room temperature derived from the fractioning of one or several natural and/or synthetic oil(s), a natural and/or synthetic resin, a silicone or silicone polymer wax, or a mixture of these.

5. The compound (1) according to any of the preceding claims, **characterized in that** said waxy matrix (3) comprises a polymethylsilsesquioxane, and is for example primarily formed by the latter.

6. The compound (1) according to any of the preceding claims, **characterized in that** said solid particles (2) have an average size comprised between 10 nm and 500 µm, preferably between 100 nm and 100 µm.

7. The compound (1) according to any of the preceding claims, **characterized in that** it is in the form of granulates, for example pellets or sticks (4), obtained by extrusion.

8. The compound (1) according to any of the preceding claims, **characterized in that** said functional agent forms at least 70 weight%, preferably at least 80 weight%, of said compound (1).

9. The compound (1) according to any of the preceding claims, **characterized in that** said functional agent and said waxy matrix (3) form together at least 95 weight%, preferably at least 97 weight%, more preferably at least 99 weight%, still more preferably all, of said compound (1).

10. The compound (1) according to any of the preceding claims, **characterized in that** it does not comprise any surfactant.

11. A method for producing a compound (1) comprising at least 50 weight% of a functional agent, **characterized in that** it comprises at least:
- a step (C) of heating up a waxy matrix (3) in order to obtain a fluid matrix (3),
- a step (T) of treating solid particles (2) during which these undergo a surface treatment causing a reduction of the capacity of absorption of the fluid matrix (3) by the treated solid particles (2), in comparison with non-treated solid particles (2) of the same kind, said treated solid particles (2) forming said functional agent,
- a step (D) of dispersing said functional agent within said fluid matrix (3),
said compound (1) being solid at room temperature and intended to be dispersed by fluidification and/or solubilization within a cosmetic and/or dermatological composition to integrate said functional agent therein.

12. The method according to the preceding claim, **characterized in that** it further comprises a step (B) of grinding said solid particles (2) intended to reduce their average size, and **in that** upon completion of said grinding step (B), said solid particles (2) have preferably an average size substantially comprised between 100 nm and 500 µm, preferably between 100 nm and 100 µm.

13. The method according to claim 11 or 12, **characterized in that** it comprises a step (E) of extruding said compound (1) for the latter to be in the form of granulates, for example pellets or sticks (4), the extrusion step (E) being preferably carried out at hot temperature, said compound (1) having during this step a temperature comprised advantageously between 60 and 80°C.

14. A method for producing a cosmetic and/or dermatological composition comprising a redispersion step (R) during which a compound (1) according to any one of claims 1 to 10 is added and then mixed with one or several cosmetically and/or dermatologically acceptable component(s).

15. The method for producing a cosmetic and/or dermatological composition according to the preceding claim, **characterized in that** said redispersion step (R) further comprises a step of solubilizing said compound (1) by one or more of said cosmetically and/or dermatologically acceptable components, and/or **in that** said redispersion step (R) further comprises a fluidification step, in which said added compound (1) is heated up at least until fluidification.
